# EUROPEAN PATENT SPECIFICATION

(11) **EP 3 038 698 B1**
(45) Date of publication and mention of the grant of the patent: **30.01.2019**
(21) Application number: 14762055.3
(22) Date of filing: 26.08.2014
(51) Int. Cl.: A61D 7/00, B05B 1/20, B05B 9/08, B65D 83/30, A61M 35/00

(54) **MEASURED DOSE DISPENSER**
SPENDER FÜR ABGEMESSENE DOSEN
DISTRIBUTEUR DE DOSE MESURÉE

(30) Priority: 30.08.2013 GB 201315529
(43) Date of publication of application: 06.07.2016
(73) Proprietor: AAN Medical Limited, Salisbury SP5 4DF (GB)
(72) Inventor: COATS, Andrew, Salisbury SP5 4DF (GB); DAVIS, Nigel, Welwyn AL6 9QA (GB); TULLOCH, Andrew, Reading RG2 9QA (GB)
(74) Representative: Beckham, Robert William
(86) International application number: PCT/GB2014/052589
(87) International publication number: WO 2015/028787

(56) References cited:
- WO-A1-2012/043075
- WO-A1-2013/139588
- US-A- 1 089 595
- US-A- 5 205 306

## Description

### Technical Field

This invention relates to dispensers for delivering doses of pharmaceuticals and other medical treatments to people. Although designed originally for humans, it can also be applied to animals, and is particularly effective in treating domestic and farm animals.

### Background Art

Provision of drugs and other medical treatments has been usually by injection, pills, and nasal sprays; more recently transdermal patches have been used to deliver a measured amount of the drug or other treatment concerned. In this specification "treatment" includes pre-treatments of persons to minimise their risk of reaction if subsequently exposed, for example, to hazardous gases.

There remains, however, an issue in ensuring the accuracy of delivery of the drug or other treatment concerned in some circumstances. In the case of transdermal treatments with patches, it is difficult to ensure that the patch continues to adhere sufficiently to enable the drug or other treatment to cross the skin barrier for a long enough time for the treatment to be effective.

A small number of drugs are available in spray-on formulations, but these require significant dexterity to apply and give very inconsistent doses due to variation in the accuracy of the user, the distance of the spray from the target and the number of times and force with which the nozzle is pressed.

WO 2012/043075 A1 relates to a liquid agent applicator comprising a nozzle attached to the tip of a syringe. The nozzle includes an arm portion having a flow passage and discharge holes formed in the inner circumferential surface of the arm portion. Each discharge hole in the nozzle is formed in the discharge direction directing the centre of the circle constituting the arm portion.

### Disclosure of Invention

According to the present invention a drug or medical treatment dispenser for the transdermal treatment of a person or animal characterised in that the dispenser (20) comprises a hoop (21) having at least one groove (22), said groove(s) 22 containing at least one tube (23) being connected at one end to a pump (31) said pump being connected to a container of treatment (32), the at least one tube (23) having one or more apertures (28) to dispense treatment in spray form towards the skin of a person or animal, the apertures (28) being inwardly directed and disposed around the inside of the hoop (28) to direct spray treatment (29) emerging from the apertures (28) onto a person's or animal's body part placed within the hoop.

Other features of the invention are set out in this specification and/or in the accompanying claims.

The invention enables the creation of a hospital or home-based device in one instance or an ambulance or battlefield device in another which can be passed over a wound and sprays on antiseptic, wound sealer etc., without the need for touch thus much reducing or eliminating the risk of cross infection.

### Brief Description of Drawings

In order that the invention may be more fully understood, examples of the invention of the invention are illustrated in the accompanying drawings in which:
Figures land 2 show perspective views of a
   dispenser according to the invention;
Figure 3 is a section on the line A-A' of figure 1;
Figure 4 is a schematic view of the dispenser of figures 1 and 2 partially sectioned on the line B-B' of figure 1;
   and
Figure 5 is similar to figure 4 but illustrates a further development of the pumped dispenser shown in figures 1 to 4.

### Detailed description of some examples of the invention

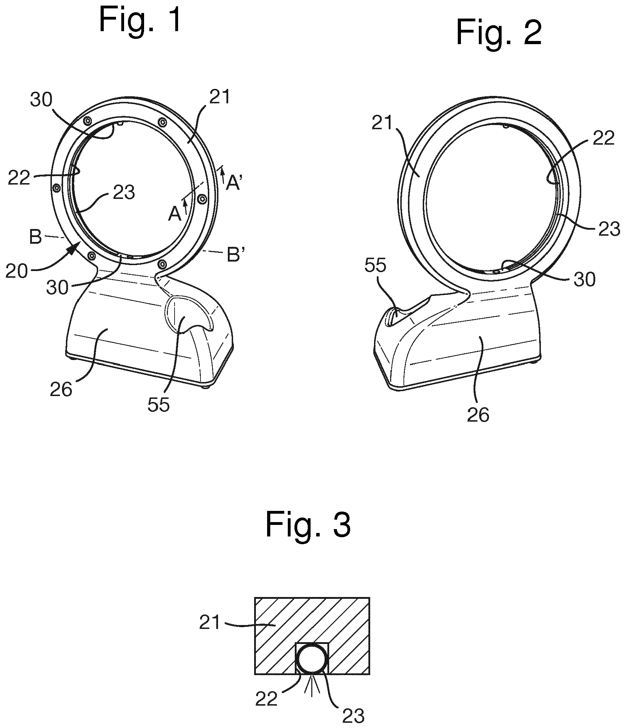

In figures 1 to 4, a dispenser 20 according to the invention comprises a circular hoop 21 of stainless steel, plastic or like material suitable for medical use. The hoop has an internal groove 22, in which is set a tube 23 made of silicon rubber or like material suitable for medical use. Separate tubes 23 extended around the inside of the left hand side of the hoop 21 and the right hand side of the hoop 21 as seen in figure 2. One end 24 of the each of the tubes 23 is joined to a manifold 25 mounted in a hollow base 26, the other ends 27 of the tubes are closed. The tubes have a plurality of apertures in the form of self-sealing slits 28 from which any fluid in the tubes under pressure is expelled as a spray. The hoop itself is mounted on the base 26. The tubes 23 are held in place at the top and bottom of the hoop 21 by plates 30. Manifold 25 is connected to the outlet 33 of an electric pump 31 mounted in the base 26. A cartridge 32 containing drugs of interest for the treatment of a particular patient is mounted through the top of the base 26, with its outlet nozzle connected to an upstanding nipple 34 on a cartridge mounting 35. The bottom of the base 26 is closed by a base plate 36. The cartridge mounting 35 is mounted on the base plate 36. A fluid connection 37 is provided in the cartridge base to a pump connector 38 also upstanding from the cartridge mounting 35. A short length of tube 39 of silicon rubber or like material suitable for medical use connects the pump connector 38 to the pump inlet 40.

Cartridge 32 is replaceable and may be subdivided to provide two or more different treatments.

The pump 31 is powered from a battery or mains source 41 through a switch 42. The switch 42 can be operated through an aperture 43 in the base plate. However, the switch in some arrangements may more conveniently be placed on the side of the base. Optionally the aperture 43 may house a flexible rubber cover which allows the switch 42 to be operated but which keeps dirt and moisture away from the inside of the base. Optionally a programmable chip 44 may be included in association with the switch 42; the chip may be programmed to limit the period in which the motor is switched on and to prevent operation of the pump 31 for a pre-set period following previous operation. The programmable chip may also be programmed to vary the pump pressure (and thus the density and spread of any spray ejected through the self-sealing slits 28.

In operation, a patient places a part of the body, say, an arm, wrist, hand, leg, ankle, foot etc., within the hoop 21. When the pump is activated by turning on the switch or operation of some other suitable activation device 42, a treatment contained in cartridge 32 is pumped by pump 31 into each of the tubes 23 around the hoop 21. The pump pressure in the tubes 23 forces liquid treatment through the self-sealing slits 28 as a spray 26 onto the patient's body part being treated.

Although developed for application to humans, there is no reason in principle why the apparatus should not be used to treat other animals in a similar way.

The devices of figures 1 to 4 have a limitation in that the size of the body part to be treated is limited by the diameter of the hoop 21.

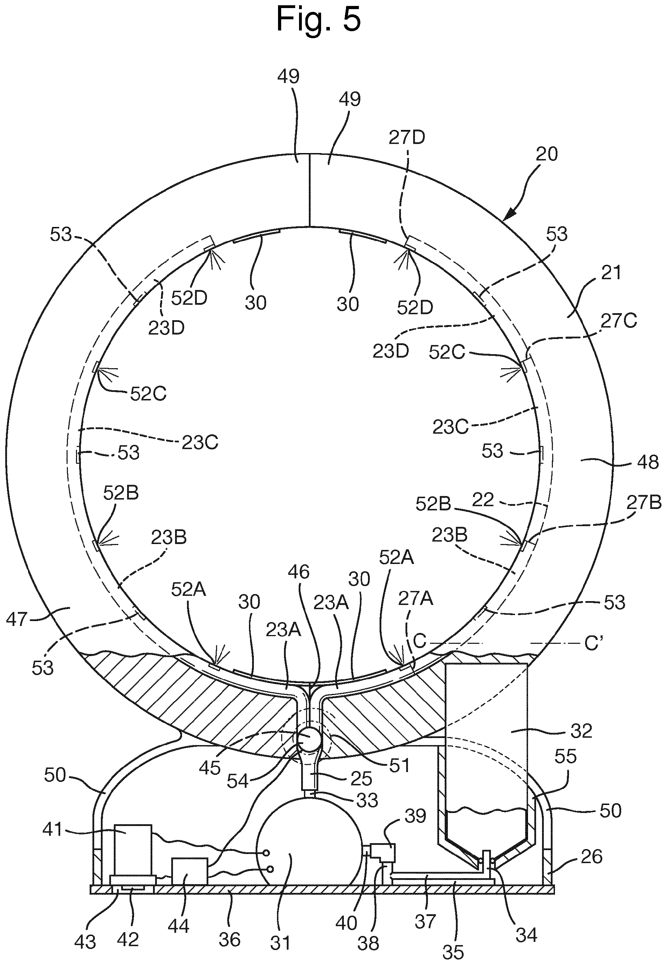

The device in figure 5 overcomes this restriction. The arrangements pumping arrangements are the same as in figure 2 and are not described again. In figure 5, the hoop 21 is formed of two semi-circular arms 47 and 48 pivoted at pivot 45 together at the lower ends of the arms (the proximal ends 46), and in the closed position butting at the other extremities of their arms (the distal ends 49). The base has a slot 50 to enable the arms 47 and 48 to open and close. Optionally there is a spring 51 associated with the pivot 45, urging the arms to close together at the distal ends 49. The ends 27 of the tubes 23, where the arms 47 and 48 butt terminate with nozzles 52 in this case. Optionally, in this configuration the self-sealing slits 28 in tubes 23 are omitted.

In the arrangement of figure 5, the arms 47 and 48 are opened and a body part inserted between their distal ends 49. The body part can be substantially larger, such a thigh, chest or shoulder than could be inserted in hoop 21 of figure 2. With the pump 31 on, and a cartridge 32 containing treatment in place on the cartridge mounting 35, treatment will be sprayed onto the body part concerned through the nozzles 52. If the pressure of the pump 31 is varied, the density, spread and range of the spray emerging from nozzles 52 can be changed.

The skilled person will be able readily to identify possible alternative configurations for the cartridge mounting and/or pump for this invention. In one very simple alternative configuration, rather than a hoop, the dispenser comprises a simple straight or hooked wand with the nozzles being directed towards the patient. The hoops or arms need not be circular or semi-circular, and could be square, elliptical or any other suitable shape.

The presence of a programmable chip 44, enables more sophisticated monitoring to include not only the passage of time since a treatment, but recording, for example, of the times of treatment, to enable medical professionals to monitor whether the patient is regularly undertaking the treatments, it could go further and set off a reminder or an alarm if a preprogrammed treatment is missed, for example. The alarm can be local to the patient, or remote at control centre, a medical centre or hospital. The chip can be programmed to dispense different amounts of treatment to different patients or to operate different treatment regimes for different patients; the patient being identified by use of different switches, or input codes or other recognition system. An IR lamp and detector, other motion sensor, may be incorporated to prevent the system from operating unless a body part was present in the hoop, or between the distal ends of the arms 47 and 48 of figure 5.

In the drawings, a battery 41 is used, this could be a long life conventional battery of a rechargeable battery such as an Li-ion battery; or the battery replaced by a mains supply stepped down to a safe voltage by a transformer, with a rechargeable battery back-up - for many domestic applications this may be the preferred option as it avoids loss of power as a result of batteries being discharged. For field operations this would not be practicable and battery power would be used.

A drug or medical treatment as described can be provided with an automatic dispensing system on detection of a body part. Furthermore, or as an alternative, such a drug or medical treatment dispenser can be provided with an automatic detection system to identify that a body part inserted was the correct body part of the correct patient, preventing activation unless the correct body part of the correct patient was inserted. Such systems could use patient recognition control, for example, by eye recognition, fingerprint recognition or RFID tag, and/or the device could gather and hold calibration data (using infra-red, ultrasound or laser) to know the body part inserted was indeed the correct part of the correct patient to prevent wrongful administration. Further it can ensure that the patient behaved correctly and did not avoid treatment by inserting another object into the device. Such a pattern recognition device could also be deployed to prevent treatment of the wrong part of the body.

It can be seen that the device is easy to use for arthritis and other sufferers with limited movement - they simply slip in the finger, wrist etc. into the hoop and a measured dose of pain killer or anti-arthritis treatment is administered via the skin.

In the examples, apertures in the form of both nozzles and self-sealing slit have been described. It would also be possible to use apertures in the form of holes in tubes 23, although these may be prone to leakage. In the tubes 23 nozzles can be used instead of the self-sealing slits.

Examples of applications of the device include spray treatments of arthritis, burns, venous ulcers, chronic wounds/ infections, eczema and other exfoliating conditions, and in trauma, eradication of MRSA from patients and ward staff/visitors, and disinfection. It can also be used for spray on wound sealing and dressings. A particular use is in treatment or applying temporary spray on dressings to patients in emergency evacuations.

The range of products that may be applied using the device includes, disinfectant/cleaning agents (such as iodine), medicinal agents (e.g. coagulants, repair stimulants, antibiotics, analgesics, anti-inflammatory agents), protective layers (to protect wound from air/infection/abrasion, to prevent loss of body fluids and loss of drugs), Immobilisers (e.g. plaster or polymer).

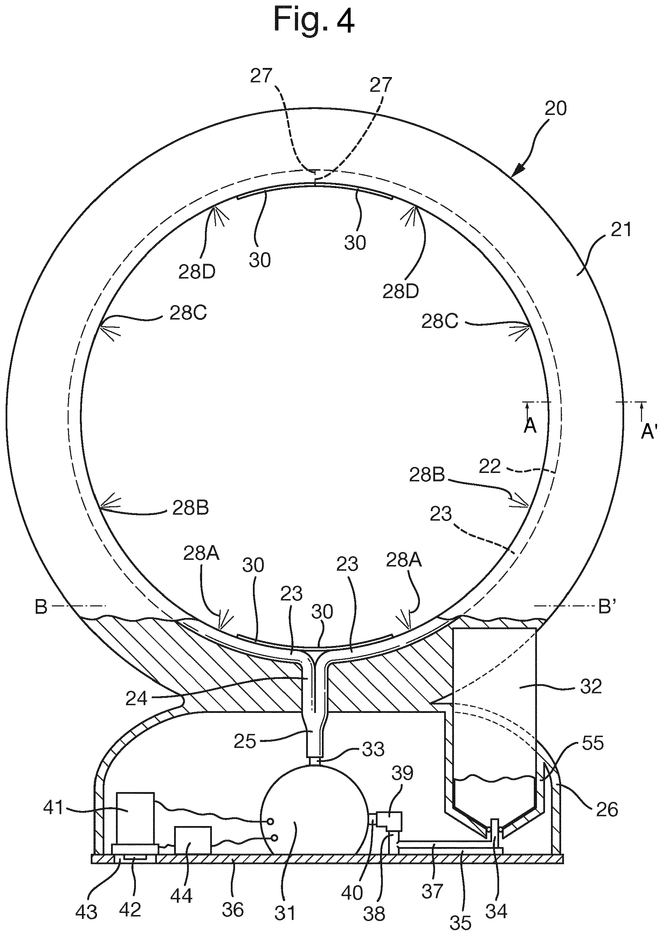

In some embodiments, each different product can carry a coding such that the spray device will apply the product in the appropriate dose and with appropriate pressure and spread of the jet. As an example, a bar code may be applied to the cartridge 32 (figures 4 or 5) containing the treatment and read by a decoder mounted on the base 36 (figures 4 or 5). The programmable chip 44 reader will take the output of the decoder and adapt the treatment regime in accordance with the coding on the cartridge 32.

## Claims

1. A drug or medical treatment dispenser for the transdermal treatment of a person or animal **characterised in that** the dispenser (20) comprises a hoop (21) having at least one groove (22), said groove(s) 22 containing at least one tube (23) being connected at one end to a pump (31) said pump being connected to a container of treatment (32), the at least one tube (23) having one or more apertures (28) to dispense treatment in spray form towards the skin of a person or animal, the apertures (28) being inwardly directed and disposed around the inside of the hoop (28) to direct spray treatment (29) emerging from the apertures (28) onto a person's or animal's body part placed within the hoop.

2. A drug or medical treatment dispenser according to claim 1 **characterised in that** the hoop has a pair of tubes (23) mounted, therein, the ends (27) of the tubes not connected to the pump (31) being closed.

3. A drug or medical treatment dispenser according to claim 2 **characterised in that** one of the tubes (23) is in one half of the hoop (21) and the other of the tubes (23) is placed in the other half of the hoop (21).

4. A drug or medical treatment dispenser according to claim 1 **characterised in that** it comprises a hoop (21) formed as two arms (47, 48), each arm having a proximal end (46) and a distal end (49), the proximal ends (46) of the arms being mounted on a pivot (45), a pair of tubes (23) one mounted in each arm, the tubes (23) at the proximal end (46) of the arms being connected to the outlet (33) of the pump (31).

5. A drug or medical treatment dispenser according to claim 4 **characterised in that** the distal ends of the tubes (27) each terminate with a nozzle (52), each nozzle (52) to direct treatment onto a body part placed between the distal ends of the arms (47,48).

6. A drug or medical treatment dispenser according to claim 4 or 5 **characterised in** having a spring (51) tending to urge the distal ends (49) of the arms (47,48) to butt together.

7. A drug or medical treatment dispenser according to any preceding claim **characterised in that** the hoop (21) is mounted on a base (26), said base containing the pump (31) with a connection (37) to the container (32) .

8. A drug or medical treatment dispenser according to claim 7 **characterised in that** the pump (32) is electrically driven and is connected to a programmable chip (44) controlling the times of treatment and to measure other parameters of the treatment.

## Patentansprüche

1. Arzneimittel- oder Medikamentenbehandlungs-Ausgabevorrichtung für die transdermale Behandlung einer Person oder eines Tiers, **dadurch gekennzeichnet, dass** die Ausgabevorrichtung (20) einen Ring (21) umfasst, der wenigstens eine Rille (22) aufweist, wobei die Rille (n) (22) wenigstens einen Schlauch (23) enthält, der an einem Ende mit einer Pumpe (31) verbunden ist, wobei die Pumpe mit einem Behälter mit Behandlungsstoff (32) verbunden ist, wobei der wenigstens eine Schlauch (23) eine oder mehrere Öffnungen (28) aufweist, um Behandlungsstoff in Sprühform in Richtung auf die Haut einer Person oder eines Tiers auszugeben, wobei die Öffnungen (28) nach innen gerichtet und um die Innenseite des Rings (28) angeordnet sind, um die Sprühbehandlung (29), die aus den Öffnungen (28) austritt, auf einen Körperteil einer Person oder eines Tiers, der innerhalb des Rings angeordnet ist, zu lenken.

2. Arzneimittel- oder Medikamentenbehandlungs-Ausgabevorrichtung gemäß Anspruch 1, **dadurch gekennzeichnet, dass** der Ring ein Paar von Schläuchen (23) darin angebracht aufweist, wobei die Enden (27) der Schläuche, die nicht mit der Pumpe (31) verbunden sind, geschlossen sind.

3. Arzneimittel- oder Medikamentenbehandlungs-Ausgabevorrichtung gemäß Anspruch 2, **dadurch gekennzeichnet, dass** einer der Schläuche (23) in einer Hälfte des Rings (21) angeordnet ist und der andere der Schläuche (23) in der anderen Hälfte des Rings (21) angeordnet ist.

4. Arzneimittel- oder Medikamentenbehandlungs-Ausgabevorrichtung gemäß Anspruch 1, **dadurch gekennzeichnet, dass** sie einen Ring (21) umfasst, der als zwei Arme (47, 48) gestaltet ist, wobei jeder Arm ein proximales Ende (46) und ein distales Ende (49) aufweist, wobei die proximalen Enden (46) der Arme an einem Gelenk (45) angebracht sind, ein Paar von Schläuchen (23), einer in jedem Arm, angebracht ist und die Schläuche (23) an dem proximalen Ende (46) der Arme mit dem Auslass (33) der Pumpe (31) verbunden sind.

5. Arzneimittel- oder Medikamentenbehandlungs-Ausgabevorrichtung gemäß Anspruch 4, **dadurch gekennzeichnet, dass** die distalen Enden der Schläuche (27) jeweils mit einer Düse (52) enden, wobei jede Düse (52) zum Lenken von Behandlungsstoff auf einen Körperteil, der zwischen den distalen Enden der Arme (47, 48) angeordnet ist, dient.

6. Arzneimittel- oder Medikamentenbehandlungs-Ausgabevorrichtung gemäß Anspruch 4 oder 5, **gekennzeichnet durch** Aufweisen einer Feder (51), die die distalen Enden (49) der Arme (47, 48) zum Aneinanderliegen zusammendrückt.

7. Arzneimittel- oder Medikamentenbehandlungs-Ausgabevorrichtung gemäß einem der vorstehenden Ansprüche, **dadurch gekennzeichnet, dass** der Ring (21) auf einer Basis (26) angebracht ist, wobei die Basis die Pumpe (31) mit einer Verbindung (37) zu dem Behälter (32) enthält.

8. Arzneimittel- oder Medikamentenbehandlungs-Ausgabevorrichtung gemäß Anspruch 7, **dadurch gekennzeichnet, dass** die Pumpe (32) elektrisch betrieben ist und mit einem programmierbaren Chip (44) verbunden ist, der die Behandlungsdauern steuert und zum Messen anderer Parameter der Behandlung dient.

## Revendications

1. Distributeur de médicament ou de traitement médical pour le traitement transdermique d'une personne ou d'un animal, **caractérisé en ce que** le distributeur (20) comprend un cerceau (21) possédant au moins une rainure (22), ladite ou lesdites rainures (22) contenant au moins un tube (23) raccordé à une extrémité à une pompe (31), ladite pompe étant raccordée à un contenant de traitement (32), l'au moins un tube (23) possédant une ou plusieurs ouvertures (28) pour distribuer un traitement sous forme pulvérisée vers la peau d'une personne ou d'un animal, les ouvertures (28) étant dirigées vers l'intérieur et disposées autour de l'intérieur du cerceau (28) afin de diriger le traitement pulvérisé (29) sortant par les ouvertures (28) sur une partie corporelle d'une personne ou d'un animal placée dans le cerceau.

2. Distributeur de médicament ou de traitement médical selon la revendication 1, **caractérisé en ce que** le cerceau possède deux tubes (23) montés en son sein, les extrémités (27) des tubes non raccordées à la pompe (31) étant fermées.

3. Distributeur de médicament ou de traitement médical selon la revendication 2, **caractérisé en ce que** l'un des tubes (23) est dans une moitié du cerceau (21) et l'autre des tubes (23) est placé dans l'autre moitié du cerceau (21).

4. Distributeur de médicament ou de traitement médical selon la revendication 1, **caractérisé en ce qu'**il comprend un cerceau (21) ayant la forme de deux bras (47, 48), chaque bras présentant une extrémité proximale (46) et une extrémité distale (49), les extrémités proximales (46) des bras étant montées sur un pivot (45), deux tubes (23) avec un monté dans chaque bras, les tubes (23) au niveau de l'extrémité proximale (46) des bras étant raccordés à l'orifice de sortie (33) de la pompe (31).

5. Distributeur de médicament ou de traitement médical selon la revendication 4, **caractérisé en ce que** les extrémités distales des tubes (27) se terminent chacune par une buse (52), chaque buse (52) permettant de diriger le traitement sur une partie corporelle placée entre les extrémités distales des bras (47, 48).

6. Distributeur de médicament ou de traitement médical selon la revendication 4 ou 5, **caractérisé en ce qu'**il comporte un ressort (51) ayant tendance à pousser les extrémités distales (49) des bras (47, 48) pour les mettre en butée l'une contre l'autre.

7. Distributeur de médicament ou de traitement médical selon l'une quelconque des revendications précédentes, **caractérisé en ce que** le cerceau (21) est monté sur une base (26), ladite base contenant la pompe (31) dotée d'un raccord (37) avec le contenant (32).

8. Distributeur de médicament ou de traitement médical selon la revendication 7, **caractérisé en ce que** la pompe (32) est électrique et est connectée à une puce programmable (44) commandant les temps de traitement et permettant de mesurer d'autres paramètres du traitement.
